# EUROPEAN PATENT APPLICATION

(11) **EP 3 403 584 A1**
(43) Date of publication of application: **21.11.2018**
(21) Application number: 18172027.7
(22) Date of filing: 14.05.2018
(51) Int. Cl.: A61B 6/00

(54) **X-RAY FLUOROSCOPIC DEVICE**

(30) Priority: 17.05.2017 JP 2017098210
(71) Applicant: Shimadzu Corporation, Kyoto 604-8511 (JP)
(72) Inventor: SHIMIZU, Tatsuya, Kyoto, 6048511 (JP); HATTORI, Atsuhiro, Kyoto, 6048511 (JP); OKAMOTO, Takeshi, Kyoto, 6048511 (JP)
(74) Representative: Müller-Boré & Partner Patentanwälte PartG mbB

(57) **Abstract**

In order to provide an X-ray fluoroscopic device that can perform X-ray fluoroscopy even when X-ray fluoroscopy is started without selecting an anatomical program, if an anatomical program has already been selected when a foot switch (10) or a hand switch (20) is turned on, an anatomical program read-out unit (83) reads out the selected anatomical program from among a plurality of anatomical programs stored in an anatomical program storage unit (81). If an anatomical program is yet to be selected when the foot switch (10) or the hand switch (20) is turned on, the anatomical program read-out unit (83) reads out a standard anatomical program stored in a standard anatomical program storage unit (82).

## Description

### FIELD OF INVENTION

The present invention relates to an X-ray fluoroscopic device that includes an X-ray irradiating unit and an X-ray detection unit configured to detect an X-ray that was irradiated from the X-ray irradiating unit, and has passed through a subject, in which X-ray fluoroscopy is performed on the subject. "X-ray fluoroscopy" as used herein also refers to a concept that includes serial radiography of taking subsequent images and storing those images at a predetermined frame rate.

### BACKGROUND ART

When performing X-ray fluoroscopy with the X-ray fluoroscopic device described above, an anatomical program (APR) suited to the type of examination and the body part is used. Here, the anatomical program is a data structure associating imaging conditions, the imaging part of the subject, an imaging method, and other types of information. A plurality of anatomical programs are configured and stored on the basis of the imaging conditions, the imaging part of the subject, the imaging method, and other types of information. When performing X-ray fluoroscopy, a plurality of anatomical programs is displayed on a display unit such as an LCD panel, and the most appropriate anatomical program is selected among the options displayed (see Patent Literature 1).

### DESCRIPTION OF PRIOR ART

### PATENT LITERATURE

[Patent Literature 1] JP 2012-143443

### SUMMARY OF INVENTION

### PROBLEM TO BE SOLVED BY INVENTION

When performing X-ray fluoroscopy using the X-ray fluoroscopic device described above, if an operator is not used to using the device, the operator may start the X-ray fluoroscopy without selecting an anatomical program. In such a case, even when the operator presses a foot switch or hand switch to start the X-ray fluoroscopy, the X-ray fluoroscopy does not start, and X-rays are not irradiated. Therefore, there is a possibility that the operator incorrectly interprets the device being faulty as the reason for the X-ray fluoroscopy not starting. In such a case, the X-ray fluoroscopy cannot be started, and this hinders smooth treatment or examination.

The present invention has been made in order to solve the above-mentioned problem, and it is an object of the present invention to provide an X-ray fluoroscopic device that can perform X-ray fluoroscopy even when X-ray fluoroscopy has started without an anatomical program being selected.

### SUMMARY OF THE INVENTION

The invention according to claim 1 is an X-ray fluoroscopic device, which is configured to perform X-ray fluoroscopy on a subject, that includes an X-ray irradiating unit, and an X-ray detection unit configured to detect an X-ray that is irradiated from the X-ray source and has passed through the subject, the X-ray fluoroscopic device including an anatomical program storage unit configured to store a plurality of anatomical programs that each correspond to an imaging part; a standard anatomical program storage unit configured to store a standard anatomical program; a selection unit configured to select an anatomical program; an operation unit that is used to perform an X-ray fluoroscopy start operation; and a control unit configured to, when the operation unit is operated after the anatomical program is selected by the selection unit, read the anatomical program selected among the plurality of anatomical programs stored in the anatomical program storage unit to start the X-ray fluoroscopy and, when the operation unit is operated without the anatomical program being selected by the selection unit, read the standard anatomical program stored in the standard anatomical program to start the X-ray fluoroscopy.

The invention according to claim 2 is the invention according to claim 1, in which the standard anatomical program is an anatomical program that takes, as an average value, an X-ray imaging condition including tube current and tube voltage that is applied to the X-ray irradiating unit, or an image processing condition for an X-ray image that is detected by the X-ray detection unit.

The invention according to claim 3 is the invention according to claim 1, in which the standard anatomical program is an anatomical program that is frequently used among the plurality of anatomical programs that each correspond to an imaging part.

The invention according to claim 4 is the invention according to any one of claims 1 to 3, in which the X-ray fluoroscopic device further includes an automatic brightness control unit configured to adjust brightness of an X-ray image that is detected by the X-ray detection unit.

With the invention according to claim 1, is it possible to perform the X-ray fluoroscopy using the standard anatomical program even when the X-ray fluoroscopy is started without selecting the anatomical program.

With the invention according to claim 2, it is possible to perform the X-ray fluoroscopy using the anatomical program with the standard X-ray fluoroscopy condition or the image processing condition.

With the invention according to claim 3, it is possible to perform the X-ray fluoroscopy using the anatomical program that is frequently used.

With the invention according to claim 4, it is possible to control the X-ray fluoroscopy image after the X-ray fluoroscopy has started so that the X-ray fluoroscopy image has the appropriate brightness using the standard anatomical program by performing the automatic brightness control.

### BRIEF DESCRIPTION OF DRAWINGS

[FIG. 1] FIG. 1 is a perspective view for illustrating an imaging unit 1 and a monitor unit 2 in an X-ray fluoroscopic device according to the present invention.
[FIG. 2] FIG. 2 is a perspective view for illustrating the imaging unit 1 in the X-ray fluoroscopic device according to the present invention.
[FIG. 3] FIG. 3 is a perspective view of a foot switch 10.
[FIG. 4] FIG. 4 is a perspective view of a hand switch 20.
[FIG. 5] FIG. 5 is a block diagram for illustrating a main control system of the X-ray fluoroscopic device according to the present invention.
[FIG. 6] FIG. 6 is a flowchart for illustrating operation when X-ray fluoroscopy is started using the X-ray fluoroscopic device according to the present invention.

### DESCRIPTION OF EMBODIMENTS

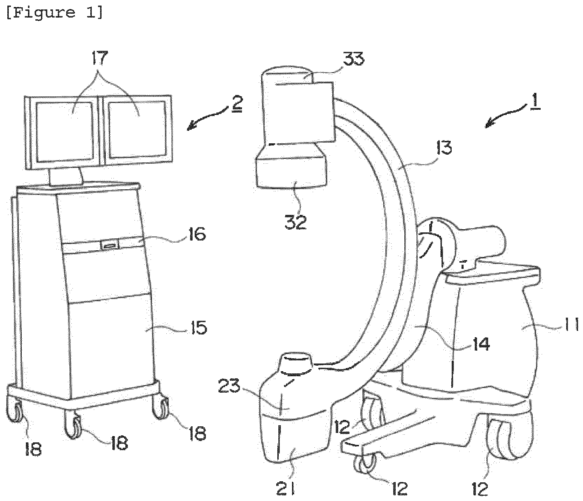

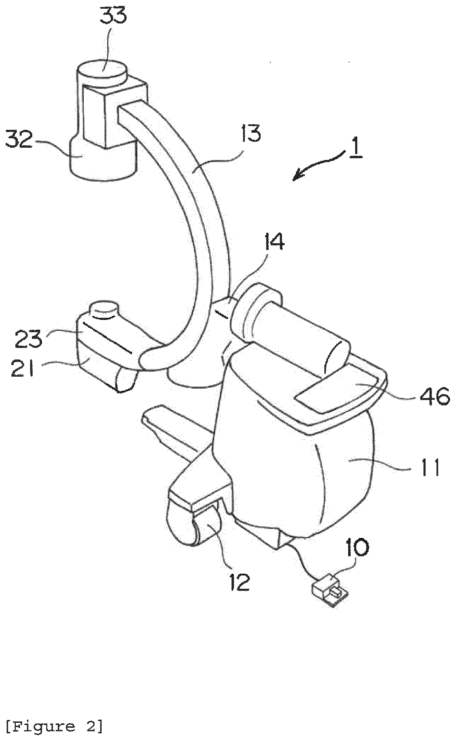

An embodiment of the present invention is described below with reference to the drawings. FIG. 1 is a perspective view for illustrating an imaging unit 1 and a monitor unit 2 in an X-ray fluoroscopic device according to the present invention. FIG. 2 is a perspective view for illustrating the imaging unit 1 in the X-ray fluoroscopic device according to the present invention.

The X-ray fluoroscopic device is a surgical X-ray fluoroscopic device that is moved to an operating room when used to perform X-ray fluoroscopy when performing surgery or the like at a surgical department. The imaging unit 1 in the X-ray fluoroscopic device includes a body 11 that can be moved by a plurality of wheels 12. In addition, the imaging unit 1 in the X-ray fluoroscopic device includes an X-ray irradiating unit that includes an X-ray tube 21, and a collimator 23 that limits an irradiation area of an X-ray irradiated from the X-ray tube 21 to form an X-ray irradiated area; an X-ray detecting unit that includes an image intensifier (I.I.) 32 configured to detect and visualize the X-ray that was irradiated from the X-ray tube 21 and that has passed through a patient, who is a subject, and a camera 33 configured to take the image visualized by the image intensifier 32; and a C-arm 13 configured to support the X-ray irradiating unit and the X-ray detection unit.

The C-arm 13 has an arc shape, and supports both the X-ray irradiating unit and the X-ray detection unit. The C-arm 13 is supported by an arm supporting portion 14 in a slideable manner. In addition, the arm supporting portion 14 is supported by the body 11 so as to be moveable in horizontal and vertical directions. The C-arm 13 can be moved by an operator grasping and operating a handle or the like (not shown).

As illustrated in FIG. 2, an LCD touch panel 46 is installed on an upper surface portion of the body 11 of the imaging unit 1. A plurality of operation switches that indicate operation items necessary for X-ray fluoroscopy and an X-ray fluoroscopy image are displayed on the LCD touch panel 46. In addition, as illustrated in FIG. 2, the body 11 of the imaging unit 1 is connected to a foot switch 10 that is used when starting the X-ray fluoroscopy. The foot switch 10 functions as an operation unit used for performing a start operation of the X-ray fluoroscopy.

A monitor unit 2 in the X-ray fluoroscopic device includes a monitor 17 configured to display an X-ray image on the basis of an X-ray detected by the X-ray detection unit in the imaging unit 1, and an input unit 16 formed of a keyboard or the like that can be stored in cart body 15. Similar to the imaging unit 1, the monitor unit 2 in the X-ray fluoroscopic device can move due to the action of a plurality of wheels 18.

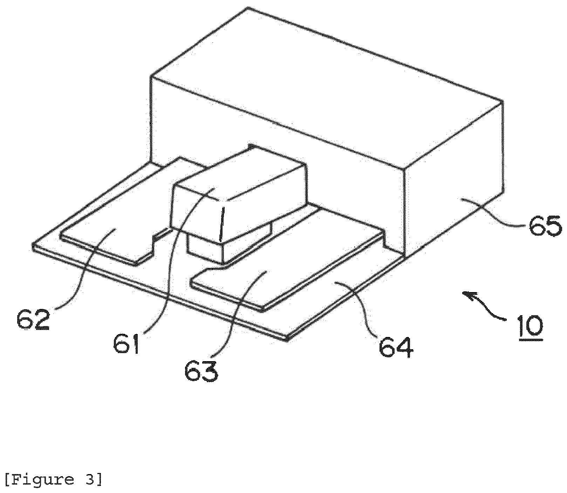

FIG. 3 is a perspective view of the above-mentioned foot switch 10.

The foot switch 10 includes a base plate 64, three switch units 61, 62, 63 that are installed on the base plate 64, and a casing 65 that houses electrical components. Among the three switch units 61, 62, 63, the switch unit 61 is used when exposing the X-ray irradiated from the X-ray source, and the switch portions 62 and 63 are used for other operations. The foot switch 10 functions as an operation unit according to the present invention that is used for a start operation of the X-ray fluoroscopy.

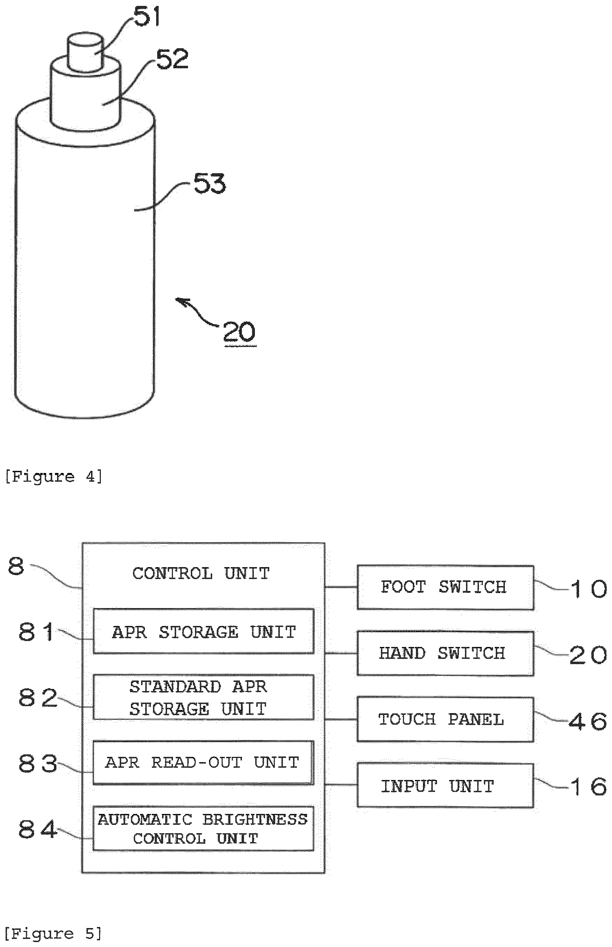

FIG. 4 is a perspective view of a hand switch 20.

The hand switch 20 is sometimes used in place of the foot switch 10 as the operation unit that is used for the X-ray fluoroscopy start operation. The hand switch 20 includes a first switch 51 and a second switch 52 that are installed on an upper portion of a body 53. The X-ray emission is prepared when the first switch 51 is pressed, and performed when the second switch 52 is pressed.

As the operation unit used to perform the X-ray fluoroscopy start operation, the LCD touch panel 46 in the imaging unit 1 or the input unit 16 in the monitor unit 2 may be used apart from the above-described foot switch 10 or hand switch 20.

FIG. 5 is a block diagram for illustrating a main control system of the X-ray fluoroscopic device according to the present invention.

The X-ray fluoroscopic device according to the present invention includes a control unit 8 configured to control the entire device. The control unit 8 includes a CPU that functions as a processor configured to process logical operations, a RAM that temporarily stores data when the control unit 8 controls the device, a ROM that stores operation programs that are needed to control the device, and other components. The control unit 8 is connected to the above-mentioned foot switch 10, hand switch 20, LCD touch panel 46, and input unit 16.

The control unit 8 also includes an anatomical program storage unit 81 configured to store a plurality of anatomical programs that each correspond to an imaging part, a standard anatomical program storage unit 82 configured to store a standard anatomical program, an anatomical program read-out unit 83 configured to read out a selected anatomical program, and an automatic brightness control unit 84. The automatic brightness control unit 84 is a unit that is used to perform automatic brightness control, which is referred to as auto brightness control (ABC), on a fluoroscopic image. Through use of this automatic brightness control, it is possible to automatically control the brightness of the fluoroscopic image to make the fluoroscopic image easier to visually recognize.

In one embodiment, the standard anatomical program stored in the standard anatomical program storage unit 82 takes an X-ray imaging condition including tube current and tube voltage that is applied to the X-ray tube 21 in the X-ray irradiating unit as an average value. Alternatively, the standard anatomical program is an anatomical program that takes an image processing condition for an X-ray image that is detected by the X-ray detection unit, as an average value. In addition, in another embodiment, the standard anatomical program stored in the standard anatomical program storage unit 82 is the anatomical program among the plurality of anatomical programs that each correspond to an imaging part that is used the most in the X-ray fluoroscopic device, or at a hospital or other facility at which the X-ray fluoroscopic device is used.

The LCD touch panel 46 in the imaging unit 1 and the input unit 16 in the monitor unit 2 are also used as a selection operation unit when the anatomical program to be used for the X-ray fluoroscopy is selected.

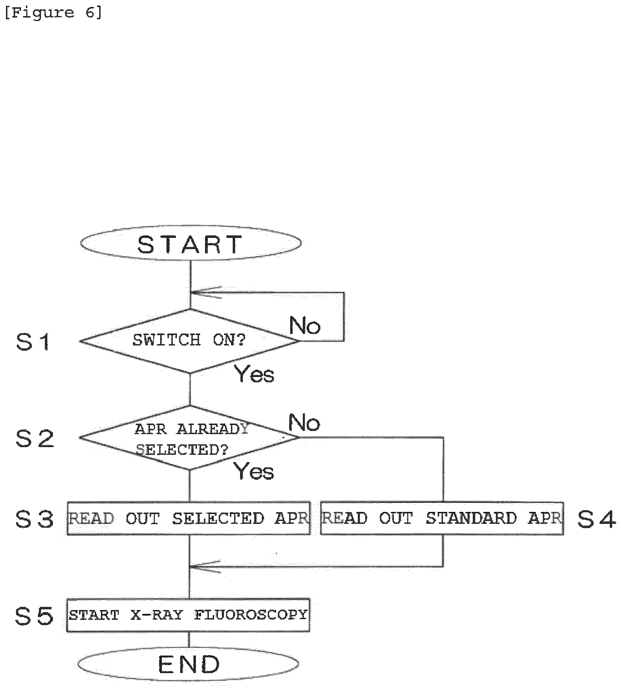

Next, operation when the X-ray fluoroscopy is performed using the above-described X-ray fluoroscopic device is described. FIG. 6 is a flowchart for illustrating the operation when the X-ray fluoroscopy is started using the X-ray fluoroscopic device according to the present invention.

When the X-ray fluoroscopy is started, the operator waits for an operation, which is the X-ray fluoroscopy start operation, that turns on the foot switch 10 illustrated in FIG. 3 or the hand switch 20 illustrated in FIG. 4 (Step S1). Then, when the foot switch 10 or the hand switch 20 turns on, the operator determines whether or not an anatomical program has already been selected beforehand (Step S2).

If an anatomical program has already been selected when the foot switch 10 or the hand switch 20 turns on, the anatomical program read-out unit 83 reads out the selected anatomical program from among the plurality of anatomical programs stored in the anatomical program storage unit 81 (Step S3). On the other hand, if an anatomical program is yet to be selected when the foot switch 10 or the hand switch 20 turns on, the anatomical program read-out unit 83 reads out the standard anatomical program stored in the standard anatomical program storage unit 82 (Step S4).

Then, then X-ray fluoroscopy starts (Step S5). At this time, if the preselected anatomical program is read out, the X-ray fluoroscopy is performed based on the imaging conditions that correspond to that anatomical program.

On the other hand, if the standard anatomical program is selected, the X-ray fluoroscopy is performed with the X-ray condition and the image processing condition as an average value, or based on the most frequently used imaging condition. At this time, because the standard anatomical program is selected, a somewhat appropriate X-ray fluoroscopy image can be obtained. Then, the automatic brightness control unit 84 adjusts the brightness of the X-ray image. With this configuration, the X-ray fluoroscopy can be performed under the most appropriate imaging conditions. Even in this case, because an average anatomical program is already selected, the time it takes to achieve an optimal X-ray image when adjusting the brightness with the automatic brightness control unit 84 can be shortened.

In the above-described embodiment, there is described a case in which the present invention has been applied to a surgical X-ray fluoroscopic device, but the present invention may also be applied to, for example, various types of X-ray fluoroscopic devices that perform X-ray fluoroscopy, including a fluoroscopic imaging table.

In addition, apart from the foot switch 10 or the hand switch 20, the LCD touch panel 46 in the imaging unit 1 or the input unit 16 in the monitor unit 2 may be used as the operation unit used for performing the X-ray fluoroscopy start operation, or another switch may be used.

### REFERENCE SYMBOLS LIST

- 1: imaging unit
- 2: monitor unit
- 8: control unit
- 10: foot switch
- 16: input unit
- 20: hand switch
- 21: X-ray tube
- 22: collimator
- 32: image intensifier
- 33: camera
- 43: LCD touch panel
- 81: anatomical program storage unit
- 82: standard anatomical program storage unit
- 83: anatomical program read-out unit
- 84: automatic brightness control unit

## Claims

1. An X-ray fluoroscopic device, which is configured to perform X-ray fluoroscopy on a subject, that comprises an X-ray irradiating unit, and an X-ray detection unit configured to detect an X-ray that is irradiated from the X-ray source and has passed through the subject, the X-ray fluoroscopic device comprising:
an anatomical program storage unit configured to store a plurality of anatomical programs that each correspond to an imaging part;
a standard anatomical program storage unit configured to store a standard anatomical program;
a selection unit configured to select an anatomical program;
an operation unit that is used to perform an X-ray fluoroscopy start operation; and
a control unit configured to, when the operation unit is operated after the anatomical program is selected by the selection unit, read the anatomical program selected among the plurality of anatomical programs stored in the anatomical program storage unit to start the X-ray fluoroscopy and, when the operation unit is operated without the anatomical program being selected by the selection unit, read the standard anatomical program stored in the standard anatomical program to start the X-ray fluoroscopy.

2. The X-ray fluoroscopic device according to claim 1, wherein the standard anatomical program is an anatomical program that takes, as an average value, an X-ray imaging condition including tube current and tube voltage that is applied to the X-ray irradiating unit, or an image processing condition for an X-ray image that is detected by the X-ray detection unit.

3. The X-ray fluoroscopic device according to claim 1, wherein the standard anatomical program is an anatomical program that is frequently used among the plurality of anatomical programs that each correspond to an imaging part.

4. The X-ray fluoroscopic device according to any one of claims 1 to 3, wherein the X-ray fluoroscopic device further comprises an automatic brightness control unit configured to adjust brightness of an X-ray image that is detected by the X-ray detection unit.
